# EUROPEAN PATENT SPECIFICATION

(11) **EP 3 130 287 B1**
(45) Date of publication and mention of the grant of the patent: **11.05.2022**
(21) Application number: 15776738.5
(22) Date of filing: 31.03.2015
(51) Int. Cl.: A61B 5/11, G01L 9/00, G01B 7/16, A61B 5/00

(54) **SENSOR DEVICE**
SENSORVORRICHTUNG
DISPOSITIF CAPTEUR

(30) Priority: 09.04.2014 JP 2014080054
(43) Date of publication of application: 15.02.2017
(60) Divisional application: 21167223.3
(73) Proprietor: Bando Chemical Industries, Ltd., Kobe-shi, Hyogo 650-0047 (JP)
(72) Inventor: OTAKA, Hideo, Kobe-shi, Hyogo 650-0047 (JP); YONEZAWA, Masaya, Kobe-shi, Hyogo 650-0047 (JP); BESSHO, Yusuke, Kobe-shi, Hyogo 650-0047 (JP)
(74) Representative: Sajda, Wolf E.
(86) International application number: PCT/JP2015/060149
(87) International publication number: WO 2015/156174

(56) References cited:
- EP-A2- 2 154 503
- WO-A1-2014/050245
- JP-A- H0 433 641
- JP-A- 2011 501 678
- US-A1- 2011 006 787
- US-A1- 2013 257 787
- US-B2- 7 578 195

## Description

### TECHNICAL FIELD

The present invention relates to a sensor device.

### BACKGROUND ART

In the field of rehabilitation, measurements have been on a daily basis about, for example, the momentum of a patient in the state of a motor paralysis, such as partial paralysis or hemiplegia, or the movable quantity or movable range of, for example, his/her joint, or about the heart rate or respiratory rate of a patient when the patient is in training or is staying in bed.

In the medical field also, the heart rate or respiratory rate of a patient, or an elderly person who requires nursing has been monitored on a daily basis.

As a method for measuring the momentum of a patient or the movable quantity or movable range of, for example, his/her joint, the following methods have been conventionally adopted: a method using a ruler, a method using a goniometer, and a method using a myoelectric sensor.

Although these methods make it possible to measure the degree of a bending of his/her elbow joint and knee joint, there are a large number of his/her regions which are not easily measured by the methods, such as his/her shoulder bone motion, his/her buttock motion, and his/her expression motion.

As a method for measuring a larger motion of a body, suggested is also a measuring method using a motion capture (see, for example, Patent Literature 1). However, the motion-capture-using method requires a large system as a whole of a measuring system; thus, it is difficult to carry the measuring system portably, and further it is complicated to make a preparation before the measurement. Furthermore, this method has a problem of making it impossible to measure the motion of a region behind the photographing device (camera).

As a method for monitoring the heart rate or respiratory rate of, for example, a patient with the passage of time, for example, Patent Literature 2 suggests a method using an capacitive pressure sensor formed by locating a pair of stretchable electro-conductive cloth pieces, respectively, on both surfaces of a sheet-like dielectric elastically deformable in all directions.

Patent Literature 3 relates to a capacitive sensor includes a dielectric layer made of an elastomer and a pair of electrodes arranged via the dielectric layer, and detects deformation on the basis of electrostatic capacity variation between the pair of electrodes. The pair of electrodes contain an elastomer and conductive fillers filled into the elastomer, are expandable and contractible in accordance with deformation of the dielectric layer, and exhibit little conductivity variation even when the pair of electrodes expand and contract. At least one of the dielectric layer and the electrodes is formed by a printing method using a dielectric layer coating containing a formation component of the dielectric layer or an electrode coating containing a formation component of the electrode.

Patent Literature 4 relates to an apparatus including a capacitance touch sensor arrangement configured to have a variable capacitance that varies when a user finger touches the capacitance touch sensor arrangement; and at least one variable resistor sensor integrated within the capacitance touch sensor arrangement wherein the variable resistor sensor has a variable resistance that varies with a sensed parameter; the apparatus including an input configured to receive an input signal including a time varying component and an output configured to provide an output signal that depends upon both the capacitance of the capacitance touch sensor arrangement and the resistance of the variable resistance sensor.

Patent Literature 5 relates to a dynamic quantity detecting member including a base substrate of which a part or the whole including a contact portion is deformed in accordance with pressing of a contact object and of which an original shape is recovered when the pressing of the contact object disappears; electrodes serving as displacement electrodes of which the plurality of electrodes are fixed to a surface or inside of the base substrate and of which at least one electrode is disposed in a deformable portion (which is a region deformable and displaceable during the deformation) of the base substrate; and wirings which are connected to the electrodes. During deformation, the displacement electrodes are deformed and displaced with the deformation and displacement of the deformable portion without separation from the base substrate and without damaging conductivity. The deformation and displacement of the deformable portion are detected as a variation in capacitance between the electrodes.

Patent Literature 6 relates to a flexible, resilient capacitive sensor suitable for large-scale manufacturing. The sensor comprises a dielectric, an electrically conductive layer on the first side of the dielectric layer, an electrically conductive layer on a second side of the dielectric layer, and a capacitance meter electrically connected to the two conductive layers to detect changes in capacitance upon application of a force to the detector. The conductive layers are configured to determine the position of the applied force. The sensor may be shielded to reduce the effects of outside interference.

### LIST OF CITATIONS

### [PATENT LITERATURES]

Patent Literature 1: WO 2005/096939
Patent Literature 2: Japanese Unexamined Patent Application Publication JP 2005-315 831 A
Patent Literature 3: EP 2 154 503 A2
Patent Literature 4: US 2013/257787 A1
Patent Literature 5: US 2011/006787 A1
Patent Literature 6: US 7 578 195 B2

### SUMMARY OF INVENTION

### TECHNICAL PROBLEM

However, according to the method described in Patent Literature 2, a person is not easily measured in the state of moving his/her body, for example, at time of being in rehabilitation training since the capacitive pressure sensor is laid below a human body lying down.

Moreover, the capacitive pressure sensor is a sensor for measuring a change in the capacitance of a portion of the sensor by a deformation of its dielectric layer in the thickness direction thereof. Accordingly, a deformation of the dielectric layer in the plane direction is not originally measured with ease which makes it impossible to measure the motion state of the body.

### SOLUTION TO THE PROBLEM

Under such situations, the present invention has been created and provides a capacitive sensor device capable of tracing a deformation of a surface of the living body, on the basis of an entirely different technical idea. The sensor device of the present invention is outlined in independent claim 1. Advantageous further developments of the invention are set forth in the dependent claims.

### ADVANTAGEOUS EFFECTS OF THE INVENTION

In the sensor device of the present invention, the sensor element, which has a dielectric layer comprising an elastomer composition and electro-conductive layers comprising carbon nanotubes, and which is reversibly deformable to change in the area of the top surface and the bottom surface of the dielectric layer, is configured to be bonded to a living body, thereby tracing a deformation of a surface of the living body. For this reason, even when the living body surface is largely deformed, various information pieces corresponding to the deformation of the living body surface are easily and surely measurable. Moreover, the size of the sensor device of the present invention is easily decreased from the viewpoint of the structure thereof. The decrease in the size makes it possible to carry this sensor device portably with ease.

### BRIEF DESCRIPTION OF THE DRAWINGS

- FIG. 1: is a schematic view illustrating an example of the sensor device of the present invention.
- FIG. 2A: is a perspective view that schematically illustrates an example of a sensor element in the sensor device of the present invention, and FIG. 2B is a sectional view taken on line A-A of FIG. 2A.
- FIG. 3A: is a perspective view that schematically illustrates another example of the sensor element in the sensor device of the present invention, and FIG. 3B is a sectional view taken on line B-B of FIG. 3A.
- FIG. 4: is a schematic view to describe one example of a forming apparatus used to produce a dielectric layer in the sensor device of the present invention.
- FIGS. 5A to 5D: are perspective views referred to for describing a process for producing a sensor element in Examples.
- FIG. 6A: is a schematic view illustrating a region to which the sensor element is bonded in Example 1, and FIG. 6B is a graph showing a change in the capacitance measured in Example 1.
- FIG. 7A: is a schematic view illustrating a region to which the sensor element is bonded in Example 2, and FIG. 7B is a graph showing a change in the capacitance measured in Example 2.
- FIG. 8A: is a schematic view illustrating a region to which the sensor element is bonded in Example 3, and FIG. 8B is a graph showing a change in the capacitance measured in Example 3.
- FIG. 9A: is a schematic view illustrating a region to which the sensor element is bonded in Example 4, and FIG. 9B is a graph showing a change in the capacitance measured in Example 4.
- FIG. 10: is a schematic view illustrating an inverting amplifier circuit used to measure the capacitance in Example 6.
- FIG. 11: is a schematic view illustrating a Schmitt trigger oscillation circuit used to measure the capacitance in Example 7.
- FIG. 12: is a schematic view illustrating a half-wave double voltage rectification circuit used to measure the capacitance in Example 8.

### DESCRIPTION OF EMBODIMENTS

Hereinafter, embodiments of the present invention will be described with reference to the drawings.

The sensor device of the present invention has a sensor element and a measurement instrument, and is used in the state of being bonded to a living body to be utilized to trace a deformation of a surface of the living body.

The sensor element includes a sheet-like first dielectric layer comprising an elastomer composition, and a first electrode layer and a second electrode layer each comprising an electro-conductive composition containing carbon nanotubes and formed on a top surface and a bottom surface of the first dielectric layer to be at least partially opposed to each other across the dielectric layer. The at least partially opposed portions of the first electrode layer and the second electrode layer constitute a detection portion. The sensor element is reversibly deformable to change the top surface and the bottom surface of the first dielectric layer in area.

The measurement instrument measures a change in the capacitance of the detection portion.

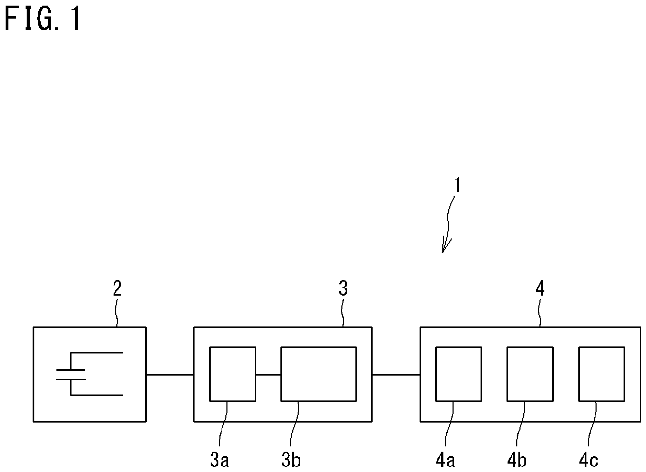

FIG. 1 is a schematic view illustrating an example of the sensor device of the present invention.

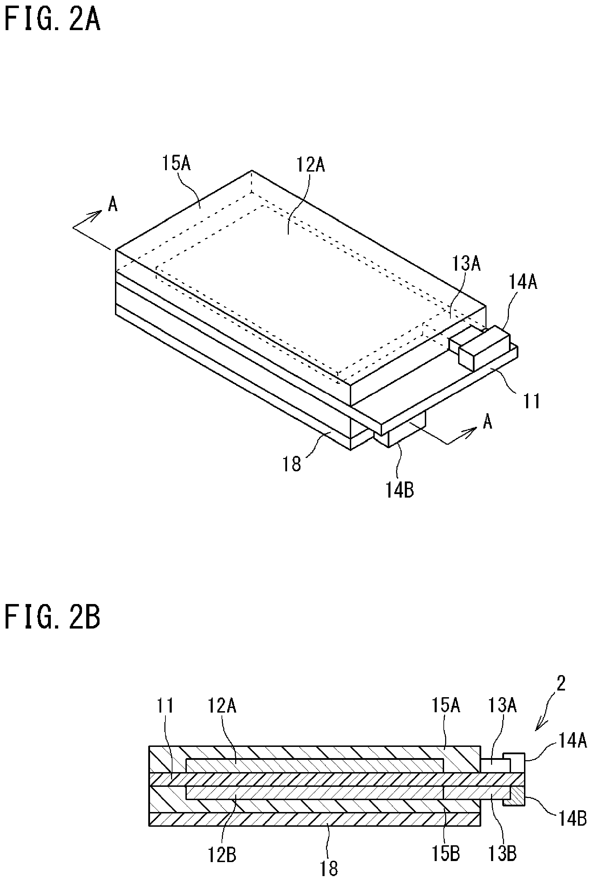

FIG. 2A is a perspective view that schematically illustrates an example of a sensor element in the sensor device of the present invention, and FIG. 2B is a sectional view taken on line A-A of FIG. 2A.

As illustrated in FIG. 1, a sensor device 1 according to the present invention has a sensor element 2 for detecting the capacitance, a measurement instrument 3 connected electrically to the sensor element 2, and an indicator 4 for displaying results measured through the measurement instrument 3.

The measurement instrument 3 has a Schmitt trigger oscillator circuit 3a for converting the capacitance C to a frequency signal F, an F/V converting circuit 3b for converting the frequency signal F to a voltage signal V, and a power source circuit (not illustrated). The measurement instrument 3 converts the capacitance C detected in a detection portion of the sensor element 2 to the frequency signal F, and further converts the frequency signal F to the voltage signal V to transmit the resultant signal to the indicator 4.

The indicator 4 has a monitor 4a, an operating circuit 4b, and a memory section 4c. The indicator 4 displays, on the monitor 4a, a change in the capacitance C that is measured by the measurement instrument 3, and further memorizes the change in the capacitance C as recorded data.

As illustrated in FIGS. 2A and 2B, the sensor element 2 has a sheet-like dielectric layer 11 comprising an elastomer composition; a top electrode layer 12A formed on a top surface (front surface) of the dielectric layer 11; a bottom electrode layer 12B formed on a bottom surface of the dielectric layer 11; a top conducting wire 13A connected to the top electrode layer 12A; a bottom conducting wire 13B connected to the bottom electrode layer 12B; a top connection portion 14A mounted on an end of the top conducting wire 13A that is opposite to a top electrode layer 12A; a bottom connection portion 14B mounted on an end of the bottom conducting wire 13B that is opposite to a bottom electrode layer 12A; a top protecting layer 15A and a bottom protecting layer 15B laminated, respectively, on the top side and the bottom side of the dielectric layer 11; and an adhesive layer 18 laminated on the bottom protecting layer 15B.

The top electrode layer 12A and the bottom electrode layer 12B have the same shape when viewed in plan. The top electrode layer 12A and the bottom electrode layer 12B are wholly opposed to each other across the dielectric layer 11. In the sensor element 2, a portion where the top electrode layer 12A and the bottom electrode layer 12B are opposed to each other constitutes a detection portion.

In the present invention, the top electrode layer and the bottom electrode layer in the sensor element are not necessarily required to be wholly opposed across the dielectric layer. These electrode layers are sufficient to be at least partially opposed to each other.

In the sensor element 2, the dielectric layer 11, the top electrode layer 12A, and the bottom electrode layer 12B correspond, respectively, to the first dielectric layer, the first electrode layer, and the second electrode layer.

In the sensor element 2, the dielectric layer 11 comprises the elastomer composition to be deformable (stretch and shrinkage) in the plane direction of this layer 11. When the dielectric layer 11 is deformed in the plane direction in the sensor element 2, the top and the bottom electrode layers 12A and 12B, and the top and the bottom protecting layers 15A and 15B (hereinafter, the two layers may be together referred to merely as the protecting layers) follow the deformation.

In the sensor device 1, with the deformation of the sensor element 2, the capacitance of the detection portion changes in correlation with the quantity of the deformation of the dielectric layer 11. Thus, the detection of the change in the capacitance makes it possible to detect the deformation quantity of the sensor element 2.

The sensor device of the present invention is a device used to trace a deformation of a surface of a living body, and is utilized in the state of being bonded to the living body. At this time, the sensor element may be bonded directly to the living body surface, or may be bonded indirectly to the living body surface to interpose, therebetween, a covering member for covering the living body surface, such as clothing, a supporter, or a bandage.

When the capacitance of the detection portion is measured in the sensor device of the present invention in the state that the sensor element is bonded to a living body, the value of the capacitance of the detection portion is changed in accordance with a deformation of a surface of the living body. Accordingly, the deformation of the living body surface can be traced on the basis of the change in the capacitance. From results of the tracing, various pieces of information can be gained.

Hereinafter, a description will be made about use embodiments of the sensor device.

### Case of Bonding Sensor Element Directly to Living Body Surface

The sensor device of the present invention is usable in the state that the sensor element is bonded directly to a living body surface, such as a skin.

In this case, the sensor element follows a deformation (such as stretching/withering, or swelling/contraction) of the living body surface to be stretched and shrunken. Thus, the capacitance of the detection portion is changed in accordance with the quantity of the deformation of the living body surface. For this reason, by measuring the capacitance of the detection portion, the defamation quantity of the living body surface is measurable. By measuring the deformation quantity of the living body surface, information pieces can be gained on biological activities of the living body that are correlative with the deformation of the living body surface, as well as on motions of the living body.

The sensor device of the present invention makes it possible to measure, as the biological activity information pieces, for example, the pulse rate (heart rate), the respiratory rate, and the level of respirations of any living body.

The motion information pieces of the living body that can be gained through the sensor device are not particularly limited. As far as the motion of the living body is such a motion that the living body surface is stretched and/or shrunken by the stretch and/or shrinkage of its muscle when the living body moves, the state of the motion is measurable.

Examples of the motion information pieces of the living body include the bending quantity (bending angle) of a joint when the joint is bent, a cheek motion when he/she pronounces or speaks, a motion of muscle of facial expression, a shoulder bone motion, a gluteal muscle motion, a back motion, the bending degree of waist, the swelling of breast, the degree of the contraction of thigh or calf by the contraction of its muscle, a throat motion when swallowing, a leg motion, a hand motion, a finger motion, a foot sole motion, a wink motion, and the stretchability (softness) of skin.

When the sensor device is used to measure a pulse rate (heart rate) of a living body, the heart rate can be gained by bonding the sensor element to a surface region of the living body where pulses are felt (the region is, for example, a radial artery or a carotid artery), and then continuing to measure the capacitance over a predetermined period. This is because the skin stretches and shrinks in accordance with the pulses, and the number of the stretches/shrinkages is consistent with the number of the pulses.

When the sensor device is used to measure a respiratory rate of a living body, the respiratory rate can be gained by bonding the sensor element to a breast region or some other region of a surface of the living body, and then continuing to measure the capacitance over a predetermined period. This is because the skin of the breast region stretches and shrinks in accordance with the respiration, and the number of the stretches/shrinkages is consistent with the number of the respirations.

When the sensor device is used to measure the bending quantity of a joint, the bending quantity of the region to be measured (the joint to be measured) can be gained by bonding the sensor element onto the region to be measured, and then measuring the capacitance while the region to be measured is moved. This is because in accordance with the movement of the region to be measured, the skin of the region is stretched and/or shrunken so that the bending quantity of the region to be measured can be calculated out in accordance with the quantity of the stretch/shrinkage.

According to the sensor device, the sensor element is bonded to a region around a mouth (for example, his/her cheek) and the capacitance is measured while in this element bonded state he/she speaks (or he/she tries to speak even when he/she cannot actually speak). At this time, the skin around his/her mouth is deformed in accordance with the kind of the spoken sound. Thus, in accordance with the deformation of the skin, the capacitance comes to be changed. It is therefore possible to gain a correlative relationship between the motion of the skin around his/her mouth when he/she speaks, and the value the capacitance or the changing manner of the value. In this way, for example, the following training can be attained.

For his/her muscle training of facial expression, sensor elements are bisymmetrically bonded to him/her, whereby the motion of his/her skin can be quantitatively be measured or can be made visible in real time. As a result, while viewing signal waveforms from the right and left sides, he/she can be in training to superpose these signals consciously onto each other or he/she can be in rehabilitation training for recovering his/her function to show a bisymmetrical and natural facial expression.

According to this sensor device, the sensor element is bonded to an ankle or instep, and the capacitance is measured while in this element bonded state he/she is taking an exercise, such as "stamping of his/her feet", "jumping", "standing-up on tiptoes", or "standing-still". At this time, in accordance with the leg or foot, skin is deformed. In accordance with the deformation, the capacitance comes to be changed. Thus, on the basis of the value of the capacitance, or the changing manner of the value, the motion of the leg or foot can be specified.

According to the sensor device, the sensor element is bonded to the palm or the back of a hand, and the capacitance is measured while in this element-bonded state he/she is taking an exercise such as "opening of his/her hand", "closing of his/her hand", "a raise of any finger" or "playing of rock-paper-scissors". At this time, in accordance with the motion of the hand, skin is deformed. In accordance with the motion of the skin, the capacitance comes to be changed. Thus, on the basis of the value of the capacitance, or the changing manner of the value, the motion of the hand can be specified.

As described above, according to the use of the sensor device of the present invention in the state of bonding its sensor element on a surface of a living body such as a skin thereof, various biological activity information pieces and living body motion information pieces can be measured.

When the sensor device is used to measure any information piece on a biological activity or a motion of any one out of plural living bodies, it is preferred to gain, as a proofreading information piece beforehand, a relationship between the kind of the motion and the value of the capacitance or the changing manner of the value for each of the living bodies to be measured. This is because a precise measurement can be made even when the living bodies have a difference between any two thereof.

### Case of Bonding Sensor Element Indirectly to Living Body Surface to Interpose Covering Member Therebetween

The sensor device of the present invention is also usable in the state of bonding the sensor element to a living body surface to interpose a covering member therebetween.

Examples of the covering member include clothing, a support, a bandage, a taping tape. The clothing is preferably clothing of such a type that when a person puts on the clothing, the clothing adheres closely to his/her skin. Specific examples thereof include underwears for training, and swimming wears.

Also when the sensor device is used in the state of bonding the sensor element to a living body surface to interpose the covering member therebetween, biological activity information pieces and living body motion information pieces can be gained in the same manner as when the sensor element is used in the state of being bonded directly to the living body surface.

Furthermore, when the sensor element is used in the state of being bonded to a living body surface to interpose a covering member therebetween, an information piece on a deformation of the covering member can be measured.

For example, when a person bonds the sensor element to his/her under wear for training and in this state he/she takes an exercise, the cloth texture of the under wear for training follows the motion of his/her body to be stretched or recovered into an original state thereof. Thus, the cloth texture is deformed. Accordingly, in accordance with the deformation (stretch and shrinkage) of the cloth texture, the capacitance comes to be changed. Thus, on the basis of the value of the capacitance, or the changing manner of the value, the deformation of the under wear (covering member) for training can be measured.

In this way, according to the use of the sensor element in the sensor device of the present invention in the state of being bonded to a living body surface to interpose the covering member therebetween, the information pieces on the deformation of the covering member can be gained as well as biological activity information pieces and living body motion information pieces.

The sensor device of the present invention may have plural sensor elements. In this case, the sensor device may gain information pieces of the same kind simultaneously at different regions of a living body, or may gain information pieces of different kinds simultaneously.

When the sensor device has two or more sensor elements as described above, for example, the sensor elements are bisymmetrically bonded to a body (for example, the instep of his/her right foot and that of his/her left foot) and in this state he/she stamps his/her feet. In this way, the balance between the respective motions of his/her right and left legs can be measured.

For example, when a person bonds the sensor elements, respectively, to his/her right and left ankles and knee joints, and hip joint and then walks in this state, measurements can be made about the balance between the respective motions of his/her right and left legs, and the bending quantity and the motion rhythm of each of his/her mobile regions. Furthermore, when he/she uses the sensor device together with an existing walking measurement instrument such as a pressure distribution sensor product for a shoe shape or a mat shape, he/she can gain higher-level information pieces on his/her walking motion.

These information pieces are useful for information pieces for deciding a menu of sports training or rehabilitation training.

In the sensor device of the present invention, the sensor element may have not only a first dielectric layer, and first and second electrode layers formed, respectively, on both surfaces of the first dielectric layer, but also a second dielectric layer and a third electrode layer.

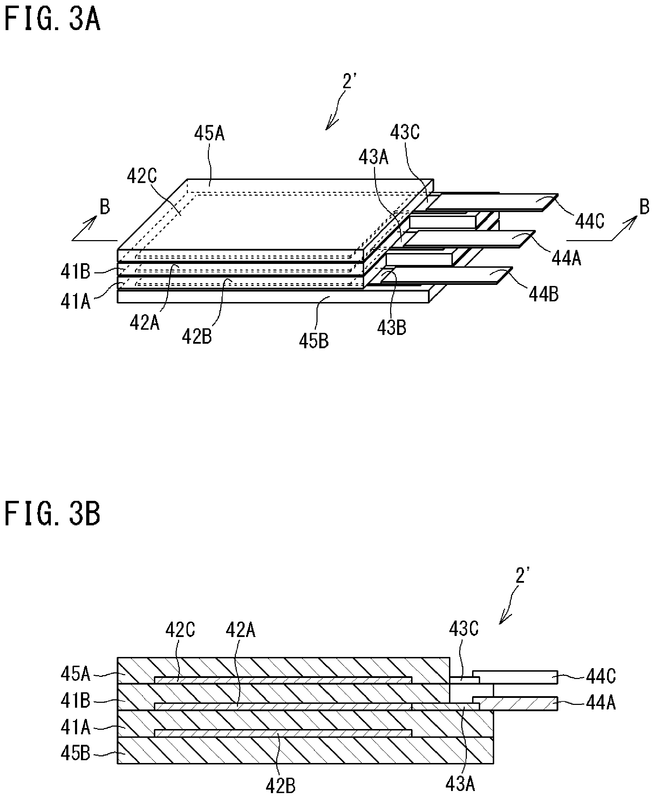

FIG. 3A is a perspective view that schematically illustrates another example of the sensor element included as a member of the sensor device of the present invention. FIG. 3B is a sectional view taken on line B-B of FIG. 3A.

A sensor element 2' illustrated in FIGS. 3A and 3B has a sheet-like first dielectric layer 41A comprising an elastomer composition; a first electrode layer 42A formed on a top surface of the first dielectric layer 41A; a second electrode layer 42B formed on a bottom surface of the first dielectric layer 41A; a second dielectric layer 41B laminated on the top side of the first dielectric layer 41A to cover the first electrode layer 42A; and a third electrode layer 42C formed on a top surface of the second dielectric layer 41B.

Furthermore, the sensor element 2' further has a first conducting wire 43A connected to the first electrode layer 42A; a second conducting wire 43B connected to the second electrode layer 42B; a third conducting wire 43C connected to the third electrode layer 42C; a first connection portion 44A mounted on an end of the first conducting wire 43A that is opposite to a first electrode layer 42A; a second connection portion 44B mounted on an end of the second conducting wire 43B that is opposite to a second electrode layer 42B; a third connection portion 44C mounted on an end of the third conducting wire 43C that is opposite to a third electrode layer 42C. Furthermore, in the sensor element 2', a bottom protecting layer 45B and a top protecting layer 45A are laminated, respectively, on the bottom side of the first dielectric layer 41A and the top side of the second dielectric layer 41B.

The first electrode layer 42A to the third electrode layer 42C have the same shape when viewed in plan. The first electrode layer 42A and the second electrode layer 42B are wholly opposed to each other across the first dielectric layer 41A. The first electrode layer 42A and the third electrode layer 42C are wholly opposed to each other across the second dielectric layer 41B. In the sensor element 2', a detection portion is not only a portion where the first electrode layer 42A and the second electrode layer 42B are opposed to each other, but also a portion where the first electrode layer 42A and the third electrode layer 42C are opposed to each other.

The capacitance of the detection portion is the sum of the capacitance of the portion where the first electrode layer 42A and the second electrode layer 42B are opposed to each other, and the capacitance of the portion where the first electrode layer 42A and the third electrode layer 42C are opposed to each other.

In the same way as the sensor element 2 illustrated in FIGS. 2A and 2B, the sensor element 2' may have an adhesive layer.

According to a sensor device having such a sensor element, measurement accidental errors based on noises are excluded so that a change in the capacitance can be more precisely measured. About this matter, a description will be made in some more detail.

The sensor device of the present invention is used in the state of bonding its sensor element to a living body, and is a sensor device for tracing a defamation of a surface of the living body. When the sensor element is bonded to the living body, the contact or approach itself of the sensor element to the living body causes noises not only when the living body surface directly contacts any one of the electrode layers but also when the living body surface contacts the electrode layer to interpose any one of the protecting layers therebetween since the living body surface is an electro-conductor.

Against this problem, the first and second electrode layers are appropriately connected to a measurement instrument, thereby making it possible to exclude measurement accidental errors based on the noises to measure a change in the capacitance precisely. Furthermore, a sensor element as have been illustrated in FIGS. 3A and 3B, which has a second dielectric layer and a third electrode layer as well as a first dielectric layer, a first electrode layer and a second electrode layer, makes the following possible: measurement accidental errors are excluded whether the top surface or the bottom surface of the sensor element is bonded to a living body, or measurement accidental errors are excluded which are based on noises from both the surfaces.

As a result, biological activity information pieces, living body motion information pieces, and covering member deformation information pieces can be more precisely measured. About the method for connecting the sensor element to the measurement instrument, a description will be made later.

Hereinafter, about each of members which the sensor device of the present invention has, a description will be made in detail.

### Sensor Element

### Dielectric Layer (First Dielectric Layer and Second Dielectric Layer)

The dielectric layer(s) is/are (each) a sheet-like layer comprising an elastomer composition, and is irreversibly deformable to change in the area of the top and bottom surfaces thereof. In the present invention, the top and bottom surfaces of the sheet-like dielectric layer denote the top surface of the dielectric layer, and the bottom surface thereof.

Examples of the elastomer composition include materials containing an elastomer, and other optional components used as required.

Examples of the elastomer include natural rubber, isoprene rubber, nitrile rubber (NBR), ethylene propylene rubber (EPDM), styrene butadiene rubber (SBR), butadiene rubber (BR), chloroprene rubber (CR), silicone rubber, fluorine rubber, acrylic rubber, hydrogen-added nitrile rubber, and urethane elastomer. They may be used alone or may be combined with one or more.

Among them, urethane elastomer and silicone rubber are preferably used because their permanent strain (or permanent elongation) is low. Furthermore, compared with the silicone rubber, the urethane elastomer is more preferably used because it is excellent in adhesion with carbon nanotubes.

The urethane elastomer is produced through a reaction between a polyol component and an isocyanate component. Specific examples thereof include an olefin-based urethane elastomer containing olefin-based polyol as the polyol component, an ester-based urethane elastomer containing ester-based polyol as the polyol component, an ether-based urethane elastomer containing ether-based polyol as the polyol component, a carbonate-based urethane elastomer containing carbonate-based polyol as the polyol component, and a castor oil-based urethane elastomer containing castor oil-based polyol as the polyol component. They may be used singly, or may be used in combination of two or more. Furthermore, the urethane elastomer may be composed of the above two or more polyol components.

Examples of the olefin-based polyol include EPOL (manufactured by Idemitsu Kosan Co., Ltd.).

Examples of the ester-based polyol include POLYLITE 8651 (manufactured by DIC Corporation).

Examples of the ether-based polyol include poly oxytetramethylene glycol, PTG-2000SN (manufactured by Hodogaya Chemical Co., Ltd.), polypropylene glycol, PREMINOL S3003 (manufactured by Asahi Glass Co., Ltd.), or PANDEX GCB-41 (manufactured by DIC Corporation).

The isocyanate component is not particularly limited, and a conventionally known isocyanate component can be used.

In addition, in synthesizing the urethane elastomer, agents such as a chain extender, a cross-linker, a catalyzer, and a vulcanization accelerator may be added during its reaction as required.

Furthermore, the elastomer composition may contain additive agents such as a plasticizer, an antioxidant, an age resistor and a colorant, and a dielectric filler other than elastomer.

An average thickness of the dielectric layer is preferably 10 µm to 1000 µm and more preferably 30 µm to 200 µm in view of increasing the capacitance C to improve the detection sensitivity, and in view of improving followability with respect to a measuring object.

It is preferable that the dielectric layer can be deformed such that its top and bottom surfaces area is increased from a non-elongation state by 30 % or more. When the dielectric layer having those characteristics is attached to the measuring object and used, it is favorably deformed in accordance with the deformation of the measuring object.

Here, being able to be deformed such that the area is increased by 30 % or more means that even when a load is applied and the area is increased by 30 %, the dielectric layer is not broken, and when the load is released, it is restored to its original state (that is, elastic deformation is provided).

A deformable range of the dielectric layer with an increase of the area of the top and bottom surfaces is preferably 50 % or more, more preferably 100 % or more, and still more preferably 200 % or more.

The deformable range of the dielectric layer in the surface direction can be controlled by a design (such as material or shape) of the dielectric layer.

Relative permittivity of the dielectric layer at room temperature is preferable 2 or more, and more preferably 5 or more. When the relative permittivity of the dielectric layer is less than 2, the capacitance C is low, and sufficient sensitivity as the sensor element may not be obtained.

Furthermore, Young's modulus of the dielectric layer is preferably 0.1 MPa to 10 MPa. When the Young's modulus is less than 0.1 MPa, the dielectric layer is too soft, and a high-quality process is difficult to perform, so that measurement accuracy may not be sufficiently high. Meanwhile, when the Young's modulus exceeds 10 MPa, the dielectric layer is too hard, so that the deformation of the living body surface could be prevented.

The hardness of the dielectric layer is preferably 0° to 30° when measured by type A durometer (JIS A hardness) based on JIS K 6253, or preferably 10° to 55° when measured by type C durometer (JIS C hardness) based on JIS K 7321.

When the dielectric layer is too soft, the high-quality process is hard to perform, so that measurement accuracy may not be sufficiently high. However, in the case where the dielectric layer is too hard, the deformation of the living body surface could be prevented.

When the sensor element has the first dielectric layer and the second dielectric layer, it is not necessarily essential that the two thereof are composed of an elastomer composition having the same composition. Preferably, however, the two thereof are composed of an elastomer composition having the same composition. This is because the two thereof show the same behavior when stretched and shrunken.

### Electrode layer (First Electrode layer to Third Electrode layer)

The electrode layer (including the top electrode layer and the bottom electrode layer) comprises an electro-conductive composition containing carbon nanotubes.

As the carbon nanotube, publicly known carbon nanotubes can be used. That is, the carbon nanotube may be a single-walled carbon nanotube (SWNT), a double-walled carbon nanotube (DWNT), or a multi-walled carbon nanotube (MWNT) (these are simply referred to as the multi-walled carbon nanotube in this specification). Furthermore, two or more kinds of carbon nanotubes having the different number of walls may be combined.

Furthermore, a shape (average length, fiber diameter, or aspect ratio) of each carbon nanotube is not limited in particular, and it may be appropriately selected in view of various factors such as intended use of the sensor device, electric conductivity and durability required for the sensor element, and processes and costs to form the electrode layer.

An average length of the carbon nanotubes is preferably 10 µm or more, and more preferably 50 µm or more.

Thus, when the electrode layer is formed of the carbon nanotubes having the large fiber length, excellent characteristics are provided, that is, the electric conductivity is high, and even when it is deformed (especially elongated) in accordance with the deformation of the dielectric layer, its electric resistance is hardly increased, and even when it is repeatedly stretched, its variation in electric resistance is small.

Meanwhile, when the average length of the carbon nanotubes is less than 10 µm, the electric resistance could be increased with the deformation of the electrode layer, and the electric resistance could largely vary after the electrode layer has been repeatedly stretched. Especially, when the deformation amount of the sensor element (dielectric layer) is increased, such defect is likely to be generated.

A preferable upper limit of the average length of the carbon nanotubes is 1000 µm. The carbon nanotubes having the average length exceeding 1000 µm are hard to produce and obtain at the moment. In addition, as will be described below, when carbon nanotube dispersion liquid having the average length exceeding 1000 µm are applied to form the electrode layer, a conducting path is not likely to be formed because the carbon nanotubes may not be sufficiently dispersed, so that the electric conductivity of the electrode layer may not be sufficiently high.

A lower limit of the average length of the carbon nanotubes is still more preferably 100 µm, and an upper limit thereof is still more preferably 600 µm. When the average length of the carbon nanotubes falls within the above range, the excellent characteristics can be more surely ensured at high level, that is, the electric conductivity is high, the electric resistance is hardly increased at the time of stretching, and the variation in electric resistance is small even after the repeated stretching.

A fiber length of the carbon nanotube may be measured from an image obtained by observing the carbon nanotubes with an electron microscope.

In addition, the average length of the carbon nanotubes may be obtained by calculating an average value based on ten carbon nanotube fiber lengths randomly chosen from the observed image of the carbon nanotubes.

An average fiber diameter of the carbon nanotubes is not limited in particular, and it is preferably 0.5 nm to 30 nm.

When the fiber diameter is less than 0.5 nm, the carbon nanotubes are not well dispersed, and as a result, the conducting path may not expand and the electric conductivity of the electrode layer may not be sufficiently high. Meanwhile, when it exceeds 30 nm, the number of the carbon nanotubes is reduced while a weight is the same, so that the electric conductivity may not be sufficiently high. The average fiber diameter of the carbon nanotubes is preferably 5 nm to 20 nm.

As for the carbon nanotube, the multi-walled carbon nanotube is more preferably used than the single-walled carbon nanotube.

In the case of using the single-walled carbon nanotube, even when the carbon nanotube to be used has the average length falling within the above-described preferable range, the electric resistance could become high, the electric resistance could be largely increased at the time of stretching, or the electric resistance could largely vary after the repeated stretching.

The reason for this is considered as follows. That is, the single-walled carbon nanotubes are commonly synthesized as a mixture of a metallic carbon nanotubes and a semiconductive carbon nanotubes, so that the semiconductive carbon nanotubes assumedly cause the problem that the electric resistance becomes high, the electric resistance is largely increased at the time of stretching, or the electric resistance largely varies after the repeated stretching.

Here, by separating the metallic carbon nanotubes and the semiconductive carbon nanotubes and only using the metallic single-walled carbon nanotubes having the large average length, it is considered possible to form an electrode layer having the same electric characteristics as those obtained by using the multi-walled carbon nanotubes having the large average length. However, it is very difficult to separate the metallic carbon nanotubes and the semiconductive carbon nanotubes (especially in the case where the carbon nanotubes have the large fiber length), so that a complicated process is required to separate them. Therefore, as described above, the multi-walled carbon nanotubes are preferably used as the carbon nanotubes in view of processability to form the electrode layer and costs.

The carbon nanotubes preferably have carbon purity of 99 % by weight or more. The carbon nanotubes may include a catalyst metal, a dispersant or the like in the production process of the carbon nanotubes, and when the carbon nanotubes containing a large amount of such components other than the carbon nanotube (impurities) are used, the electric conductivity could be lowered, and the electric resistance could vary.

The carbon nanotubes may be produced by a conventionally known method, and it is preferably produced by a substrate growth method.

The substrate growth method is one of CVD methods, in which the carbon nanotube is grown and produced by supplying a carbon source to a metal catalyst applied on a substrate. The substrate growth method is a suitable method for manufacturing the carbon nanotubes having a relatively long and uniform fiber length. Therefore, this method is suitable for manufacturing the carbon nanotube used in the present invention.

In the case where the carbon nanotubes are produced by the substrate growth method, the fiber length of the carbon nanotube is substantially equal to a length of growth of a CNT forest. Therefore, when the fiber length of the carbon nanotube is measured with the electron microscope, the length of growth of the CNT forest is to be measured.

The electro-conductive composition may contain a binder component other than the carbon nanotubes.

The binder component functions as a binding material, and when the binder component is contained, adhesion between the electrode layer and the dielectric layer, and the strength of the electrode layer itself can be improved. Furthermore, when the binder component is contained, the carbon nanotubes are prevented from being scattered when the electrode layer is formed by a method which will be described below, so that safety can be enhanced at the time of forming the electrode layer.

Examples of the binder component include butyl rubber, ethylene propylene rubber, polyethylene, chlorosulfonated polyethylene, natural rubber, isoprene rubber, butadiene rubber, styrene butadiene rubber, polystyrene, chloroprene rubber, nitrile rubber, polymethylmethacrylate, polyvinyl acetate, polyvinyl chloride, acrylic rubber, and styrene-ethylene-butylene-styrene block copolymer (SEBS).

Furthermore, the binding component may be a raw rubber (an unvulcanized natural rubber and an unvulcanized synthetic rubber). When the raw rubber having relatively low elasticity is used, the followability of the electrode layer with respect to the deformation of the dielectric layer can be enhanced.

It is especially preferable that the binder component is the same kind as the elastomer in the dielectric layer because the adhesion between the dielectric layer and the electrode layer can be remarkably improved.

The electro-conductive composition may further contain various additive agents other than the carbon nanotube and the binder component. Examples of the additive agents include a dispersion agent to improve dispersibility of the carbon nanotube, a cross-linker for the binder component, a vulcanization accelerator, a vulcanization aid, an age resistor, a plasticizer, a softener, and a colorant.

The electrode layer in the sensor element may be made of substantially carbon nanotubes alone. In this case also, sufficient adhesion with the dielectric layer can be provided. In this case, the carbon nanotube and the dielectric layer are strongly adhered by van der Waals force.

An amount of the carbon nanotubes contained in the electrode layer is not limited in particular as long as the electric conductivity can be provided. While the amount of the carbon nanotubes depends on the kind of the binder component when the binder component is contained, it is preferably 0.1 % by weight to 100 % by weight with respect to a total amount of the solid components in the electrode layer.

Furthermore, as the amount of the carbon nanotubes is increased, the electric conductivity of the electrode layer can be improved. Therefore, even when the electrode layer is thinned, the required electric conductivity can be ensured. As a result, it becomes easier to thin the electrode layer and ensure flexibility of the electrode layer.

An average thickness of the electrode layer is preferably 0.1 µm to 10 µm. When the average thickness of the electrode layer falls within the above range, the electrode layer can attain the more excellent followability with respect to the deformation of the dielectric layer.

Meanwhile, when the average thickness is less than 0.1 µm, the electric conductivity is not sufficiently high, and measurement accuracy of the sensor element could be lowered. Meanwhile, when the average thickness exceeds 10 µm, the sensor element becomes hard due to stiffening effect of the carbon nanotube, so that stretchability of the sensor element is lowered. As a result, when the sensor element is bonded directly to the living body surface, or is bonded indirectly to the living body surface to interpose a covering member therebetween, the deformation of the living body surface could be prevented.

In the present invention, "the average thickness of the electrode layer" can be measured, for example, with a laser microscope (such as VK-9510 manufactured by KEYENCE CORPORATION). More specifically, the electrode layer formed on the surface of the dielectric layer is scanned in a thickness direction at intervals of 0.01 µm, and its 3-dimensional shape is measured. Then, in the region having the electrode layer on the dielectric layer and the region not having the electrode layer on the dielectric layer, respective average heights are measured in rectangular regions of 200 µm × 200 µm and a difference between the average heights is regarded as the average thickness of the electrode layer.

It is not necessarily essential that the individual electrode layers (the first electrode layer to the third electrode layer), which the sensor element has, are made of an electro-conductive composition having the same composition. Preferably, however, the electrode layers are made of an electro-conductive composition having the same composition.

### Others

As seen in the examples illustrated in FIGS. 2A to 3B, in the sensor element, a first conducting wire (top conducting wire), a second conducting wire (bottom conducting wire) and a third conducting wire may be formed as required.

Each of these conducting wires is sufficient to be a member which does not hinder any deformation of the dielectric layer, and further maintains electro-conductivity even when the dielectric layer deforms. A specific example thereof is a conducting wire made of the same electro-conductive composition which any one of the electrode layers is composed of.

Furthermore, as seen in the examples illustrated in FIGS. 2A to 3B, a first connection portion (top connection portion), a second connection portion (bottom connection portion), and a third connection portion for being each connected to external conducting wires may be formed at respective ends of the above-mentioned conducting wires that are opposite to the respective electrode layer of the conducting wires, as required. Each of these connection portions may be, for example, a portion formed using such as a copper foil piece.

As seen in the examples illustrated in FIGS. 2A to 3B, in the sensor element, one or two protecting layer(s) may be laminated on the top outermost layer and/or the bottom outermost layer, as required. When the protecting layer(s) is/are laminated thereon, electro-conductive regions (such as the electrode layers) of the sensor element can be electrically insulated from a region where the sensor element is to be bonded. Furthermore, when the protecting layer(s) is/are laminated thereon, the sensor device can be heightened in strength and endurance, and the outer surface of the sensor element can be rendered a non-adhesive surface.

The material of the protecting layer is not particularly limited. The material is sufficient to be appropriately selected in accordance with properties required for the layer. As a specific example of the material, it includes the same material as that of the electrode layer.

As seen in the example illustrated in FIGS. 2A and 2B, in the sensor element, an adhesive layer may be formed on the bottom outermost layer of the sensor element. This formation makes it possible to bond the sensor element onto, for example, a living body surface to interpose the adhesive layer therebetween.

The adhesive layer is not particularly limited. The layer may be, for example, a layer made of an acrylic adhesive, a rubbery adhesive or a silicone adhesive.

The adhesive may be of a solvent type, an emulsion type, or a hot melt type. The adhesive is sufficient to be appropriately selected and used in accordance with, for example, the use manner of the sensor device. However, the adhesive layer needs to have such a flexibility that the stretch and shrinkage of the dielectric layer are not prevented.

In a case where stretching is repeated 1000 cycles in which one cycle means that the sensor element is stretched in one axial direction by 100 % from a non-elongation state and returned to the non-elongation state, the sensor element preferably has a small change rate between the electric resistance when the electrode layer is stretched by 100 % in the second cycle, and the electric resistance when the electrode layer is stretched by 100 % in the 1000th cycle (an absolute value of [electric resistance value when stretched by 100 % in the 1000th cycle - electric resistance value when stretched by 100 % in the second cycle] / [electric resistance value when stretched by 100 % in the second cycle] × 100). More specifically, it is preferably 10 % or less, and more preferably 5 % or less.

Here, the reason why the electric resistance value of the electrode layer in the second cycle instead of the first cycle is used is that a behavior of the electrode layer (fluctuation of the electric resistance) when the electrode layer is stretched from the non-elongation state for the first time (in the first cycle) considerably differs from those after the second time (second cycle). This is considered due to the fact that the state of the carbon nanotubes in the electrode layer is not stabilized until the sensor element is once stretched after it has been manufactured.

The sensor element is manufactured through the following steps. That is, the sensor element is manufactured through
(1) a step of forming the dielectric layer composed of the elastomer composition (step (1)), and
(2) a step of forming the electrode layers by applying the composition containing the carbon nanotubes and a dispersion medium, on the dielectric layer.

### Step (1)

In this step, the dielectric layer is formed of the elastomer composition.

First, a raw material composition is prepared such that an elastomer (or its raw material) is mixed with additive agents such as a chain extender, a cross-linker, a vulcanization accelerator, a catalyzer, a dielectric filler, a plasticizer, an antioxidant, an age resistor, and a colorant as required. After that, the raw material composition is formed into the dielectric layer. Furthermore, its forming method may be a conventionally known method.

More specifically, when the dielectric layer containing urethane elastomer is formed, the following method may be used.

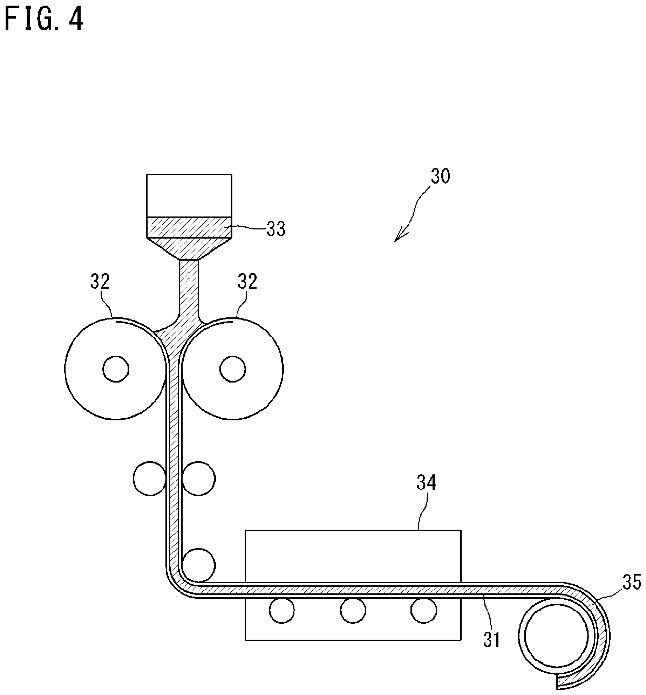

First, a mixture is prepared by measuring the polyol component, the plasticizer, and the antioxidant, and mixing and stirring them with heat for a predetermined time under reduced pressure. Then, the mixture is measured and its temperature is adjusted, and the catalyzer is added and stirred with such as an agitator. After that, a predetermined amount of the isocyanate component is added and stirred with such as the agitator, and the mixture is immediately poured in a forming apparatus illustrated in FIG. 4, and cross-linked and cured while it is sandwiched by protective films and transferred, whereby a sheet with the protective films having a predetermined thickness is obtained. After that, it is cross-linked in a furnace for a predetermined time, whereby the dielectric layer is produced.

FIG. 4 is a schematic view to describe one example of the forming apparatus used to produce the dielectric layer. A forming apparatus 30 illustrated in FIG. 4 forms a sheet-like dielectric layer 35 in such a manner that a raw material composition 33 is poured between protective films 31 made of polyethylene terephthalate (PET) and sequentially rolled out from a pair of separately disposed rolls 32 and 32, and while the raw material composition 33 is cured (cross-linked) in a sandwiched state, the raw material composition 33 is introduced into a heating apparatus 34 to be thermally cured between the pair of protective films 31.

The dielectric layer may be produced with various coating apparatuses, a general-purpose film-forming apparatus such as bar coater or doctor blade by a general film-forming method after the raw material composition has been prepared.

### Step (2)

In this step, the composition containing the carbon nanotubes and the dispersion medium (carbon nanotube dispersion liquid) is applied, and then the dispersion medium is removed in a drying process, whereby the electrode layer is formed to be integrated with the dielectric layer.

More specifically, the carbon nanotubes are added to the dispersion medium. At this time, the above-described other component such as binder component (or a raw material of the binder component) and a further dispersion agent may be added as required.

Subsequently, the components including the carbon nanotubes are dispersed (or dissolved) in the dispersion medium with a wet dispersion machine, whereby an application liquid (carbon nanotube dispersion liquid) is prepared. More specifically, the components containing the carbon nanotubes may be dispersed with an existing dispersion machine such as ultrasonic dispersion machine, jet mill, or bead mill.

Examples of the dispersion medium include toluene, methyl isobutyl ketone (MIBK), alcohol, and water. These dispersion media may be used singly, or may be used in combination of two or more thereof.

In the application liquid, a concentration of the carbon nanotubes is preferably 0.01 % by weight to 10 % by weight. When the concentration is less than 0.01 % by weight, the concentration of the carbon nanotubes is too low, and the liquid needs to be applied several times in some cases. Meanwhile, when it exceeds 10 % by weight, viscosity of the application liquid becomes too high, and the carbon nanotubes are not likely to be dispersed due to re-aggregation, so that the uniform electrode layer is difficult to form in some cases.

Subsequently, the application liquid is applied to a predetermined position on the surface of the dielectric layer by a method such as spray coating, and then dried. At this time, the application liquid may be applied after masking material has been applied to a position other than the position of the electrode layer on the dielectric layer as required.

A drying condition of the application liquid is not limited in particular, and it may be appropriately selected according to the kind of the dispersion medium and a condition of the elastomer composition.

Furthermore, the method for applying the application liquid is not limited to the spray coating. It includes such as screen printing and ink jet printing as other application methods.

After the dielectric layer and the electrode layer have been formed through the above steps (1) and (2), the conducting wires connected to the electrode layers and the connecting portions are formed as required.

The conducting wire to be connected to the electrode layer is formed by the same method as used to form the electrode layer such that the carbon nanotube dispersion liquid (application liquid) is applied to a predetermined position and dried. Furthermore, the conducting wire may be simultaneously formed with the electrode layer.

The connecting portion may be formed by attaching copper foil at the predetermined end of the conducting wire, for example.

When a sensor element having a structure as illustrated in FIGS. 3A and 3B is manufactured, two dielectric layers each composed of an elastomer composition are produced by the method in the step (1). Next, electrode layers are formed by the method in the step (2), respectively, on both surfaces of one of the dielectric layer and an electrode layer is formed on a surface of the other dielectric layer. Thereafter, the two dielectric layers, in each of which the electrode(s) is/are formed, are bonded to each other not to put any one of the electrode layers onto the other electrode layers. Thereafter, it is advisable to form conducting wires and connection portions that are each connected to the electrode layers, as required.

After the formation of the electrode layers and the optional formation of the conducting wires and the connection portions, one or two protecting layer(s) may be formed on the top outermost layer and/or the bottom outermost layer.

For the formation of the protecting layer(s), it is advisable, for example, to use the same method as used in the step (1) to produce a sheet-like product composed of an elastomer composition; cut the product into one or two pieces (each) having a predetermined size; and then laminate the piece(s) onto the electrode-layer-formed workpiece.

When a sensor element having one or more protecting layers is manufactured, the sensor element may be manufactured by forming a bottom protecting layer as a start of the production, and then laminating, thereon, constituent members (a second electrode layer, a first dielectric layer, a first electrode layer, (a second dielectric layer and a third electrode layer), and a top protecting layer) successively.

Through such steps, the sensor element can be manufactured.

The sensor element illustrated in FIGS. 2A and 2B has a single detection portion. However, the number of detection portions of the sensor element is not limited to one. Thus, the sensor element may have plural detection portions.

A specific example of the sensor element having the plurality detection portions is a sensor element in which an electrode layer in the form of rectangles as plural rows is formed, as each of a top electrode layer and a bottom electrode layer, on each of the top surface and the bottom surface of a dielectric layer, and further the rows of the top electrode layer are arranged to be perpendicular to those of the bottom electrode layer when viewed in plan. In such a sensor element, plural portions where its top electrode layer and its bottom electrode layer are opposed to each other across the dielectric layer function as detection portions, which are arranged in a lattice form.

### Measurement instrument

The measurement instrument is connected to the sensor element. The measurement instrument has a function of measuring the capacitance C of the detection portion that is changed in accordance with a deformation of the dielectric layer. The method for measuring the capacitance C may be a conventionally known method. Thus, the measurement instrument has a capacitance measuring circuit, an operating circuit, an amplifier circuit, and a power source circuit that are needed. The method (circuit) for measuring the capacitance C is, for example, a CV converting circuit (such as an LCR meter) using an automatic balanced bridge circuit, a CV converting circuit using an inverting amplifier circuit, a CV converting circuit using a half-wave double voltage rectification circuit, a CF oscillation circuit using a Schmitt trigger oscillation circuit, or a method in which a Schmitt trigger oscillation circuit is combined with an F/V converting circuit and this combination is used.

From the viewpoint of excluding the effects of noises in measurement, in the sensor device of the present invention, the sensor element is electrically connected to the measurement instrument in a manner described below.

(1-1) Case in which the sensor element is a sensor element as illustrated in FIGS. 3A and 3B, which has two dielectric layers (first and second dielectric layers) and respective electrode layers (first to third electrode layers) on both surfaces of each of the dielectric layers; and the measurement instrument is a measurement instrument using a CF oscillation circuit (such as a Schmitt trigger oscillation circuit) that is oscillated by the capacitance C and the resistance R of the detection portion, thereby measuring a change in the capacitance.

In this case, the first electrode layer is connected to the oscillation block (detecting block), and the second electrode layer and the third electrode layer are connected to the earth (i.e., the electrodes are connected to the GND of the measurement instrument).

Such a connection of the sensor element to the measurement instrument makes it possible to exclude noises even when either the top side or the bottom side of the sensor element is connected to a living body to be made close to and contactable with the living body. As a result, a change in the capacitance is more precisely measurable.

(1-2) Case in which the sensor element is a sensor element as illustrated in FIGS. 3A and 3B, which has two dielectric layers, and respective electrode layers on both surfaces of each of the dielectric layers; and the measurement instrument is a measurement instrument using a CV converting circuit such as a half-wave double voltage rectification circuit, an inverting amplifier circuit, or an automatic balanced bridge circuit. (In this case, the CV converting circuit is a CV converting circuit for passing alternating signals generated in a different block (for example, an AC applying device) through the sensor element, and then generating a voltage change by measuring or using a change in the alternating impedance of the sensor element by a change in the capacitance of the sensor element.)

In this case, the first electrode layer is connected to the detecting block, and the second electrode layer and the third electrode layer are connected to the block for generating the alternating signals.

Such a connection of the sensor element to the measurement instrument makes it possible to exclude noises even when either the top side or the bottom side of the sensor element is connected to a living body to be made close to and contactable with the living body. As a result, a change in the capacitance is more precisely measurable.

(2-1) Case in which the sensor element is a sensor element as illustrated in FIGS. 2A and 2B, which has a single dielectric layer, and respective electrode layers (top electrode layer and bottom electrode layer) on both surface of this dielectric layer; and the measurement instrument is a measurement instrument using a CF converting circuit such as a Schmitt trigger oscillation circuit.

In this case, the top electrode layer is connected to an oscillation block (detecting block) of the measurement instrument, earthing the bottom electrode layer (i.e., connecting this layer onto the GND of the measurement instrument), and further bond the sensor element to a living body to make the bottom surface side of the sensor element close to and contactable with the living body.

By bonding the sensor element to the living body in this direction and connecting the sensor element to the measurement instrument as described above, the effect of noises can be excluded. As a result, a change in the capacitance is more precisely measurable.

(2-2) Case in which the sensor element is a sensor element as illustrated in FIGS. 2A and 2B, which has a single dielectric layer, and respective electrode layers (top electrode layer and bottom electrode layer) on both surface of this dielectric layer; and the measurement instrument is a measurement instrument using a CV converting circuit such as a half-wave double voltage rectification circuit, an inverting amplifier circuit, or an automatic balanced bridge circuit.

In this case, the top electrode layer is connected to a detecting block inside the measurement instrument, connecting the bottom electrode layer to the alternating-signal-generating block, and further bond the sensor element to a living body to make the bottom surface side of the sensor element close to and contactable with the living body.

By bonding the sensor element to the living body in this direction and connecting the sensor element to the measurement instrument as described above, the effect of noises can be excluded. As a result, a change in the capacitance is more precisely measurable.

### Indicator

As seen in the example illustrated in FIG. 1, the sensor device of the present invention may have an indicator. This indicator makes it possible that in real time a user of the sensor device verifies information pieces based on a change in the capacitance C regarding, for example, living body motion information pieces. The indicator has, for example, a monitor, an operating circuit, an amplifier circuit, a power source circuit that are required for the verification.

As seen in the example in FIG. 1, the indicator may have a memory section, such as a RAM, a ROM or a HDD, for memorizing measured results of the capacitance C. When the sensor device of the present invention is used for, e.g., a person who is in sports training or rehabilitation training, the indicator makes it possible to verify, after the training, information pieces based on a change in the capacitance C regarding, for example, living body motion information pieces. For this reason, the person can check the achievement level of the training. Thus, this matter can encourage the person. Moreover, the achievement level of the training can be checked to make use of the information pieces to prepare a new training-menu.

The above-mentioned measurement instrument may have the memory section.

As the indicator, a terminal instrument, such as a personal computer, a smartphone or a tablet computer, may be used.

In the sensor device 1 illustrated in FIG. 1, the measurement instrument 3 and the Indicator 4 are connected with a wire. However, they are not necessarily connected with the wire in the sensor device in the present invention, and they may be wirelessly connected. Depending on a usage condition of the sensor device, it is sometimes more convenient that the measurement instrument and the Indicator are physically separated.

Such a sensor device of the present invention is used in the state of being bonded to a living body to trace a deformation of a surface of the living body, thereby making it possible to measure, for example, biological activity information pieces, living body motion information pieces, or covering member deformation information pieces. For this reason, the sensor device is usable in various fields, for example, to measure the heart rate or respiratory rate of any living body, to measure a momentum or physical mobile function when he/she is in sports training or rehabilitation training, to measure the stretchability of a sewn sportswear, support or the like, to evaluate the following property of a sewn sportswear, support or the like to the motion of a body, and further to assist information-transfer through conversation.

In the sensor device of the present invention, the sensor element is usable as an alternate product for an interface of a myoelectric sensor for an electrically-powered artificial hand or leg.

In the sensor device of the invention, the sensor device is usable also as an inputting terminal of an inputting interface for a serious psychosomatic handicapped person.

### EXAMPLES

Hereinafter, the present invention will be described more specifically with an example, but the present invention is not limited to the following example.

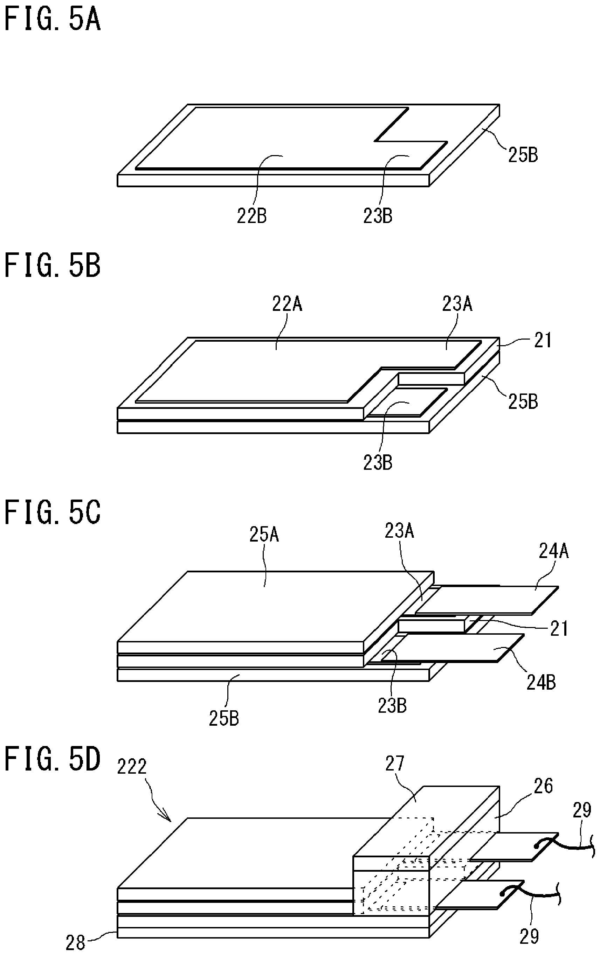

FIGS. 5A to 5D are perspective views referred to for describing a producing process of a sensor element in the examples.

Production of Adhesive-Layer-Attached Sensor Element A

### (1) Production of Dielectric Layer

To 100 parts by weight of a polyol (PANDEX GCB-41, manufactured by DIC Corp.) were added 40 parts by weight of a plasticizer (dioctyl sulfonate) and 17.62 parts by weight of an isocyanate (PANDEX GCA-11, manufactured by DIC Corp.). An agitator was used to agitate these components for 90 seconds to be mixed with each other to prepare a dielectric-layer-forming raw material composition. Next, the raw material composition was injected into a forming apparatus 30 illustrated in FIG. 4. While the composition was transported in the state of being sandwiched between protecting films 31, the composition was crosslinked and cured under conditions that the in-furnace temperature was 70 °C and the in-furnace period was 30 minutes to yield a protecting-layer-attached sheet having a predetermined thickness and wound onto a roll. Thereafter, the inside of this sheet was crosslinked in a furnace having a temperature adjusted to 70 °C for 12 hours to produce a sheet composed of a polyether type urethane elastomer. The resultant urethane sheet was cut into a piece of 14 mm × 74 mm × 70 µm thickness. Furthermore, one corner thereof was cut into a size of 7 mm × 7 mm × 70 µm thickness to produce a dielectric layer.

In addition, the elongation (%) at break and the relative permittivity of the produced dielectric layer were measured. As a result, the elongation (%) at break was 505 %, and the relative permittivity was 5.8.

The break elongation was measured based on JIS K 6251. The relative permittivity was found by measuring capacitance at a measurement frequency of 1 kHz with LCR HiTESTER (3522-50 manufactured by HIOKI E. E. CORPORATION) while the dielectric layer was sandwiched by electrodes of 20 mmφ, and then making a calculation based on an electrode area and a thickness of the measurement sample.

### (2) Preparation of Electrode Layer Material

To 30 g of methyl isobutyl ketone (MIBK) were added 30 mg of highly oriented carbon nanotubes (the number of its layers: 4 to 12; the fiber diameter: 10 nm to 20 nm; the fiber length: 150 µm to 300 µm; and the carbon purity: 99.5 %) manufactured by TAIYO Nippon Sanso Corp., which are multilayered carbon nanotubes produced by the substrate growth method, and then a jet mill (NANO JET PUL JN10-SP003, manufactured by Jokoh Co., Ltd.) was used to subject the slurry to wet dispersing treatment. The resultant was diluted 10 times to yield a carbon nanotube dispersion liquid having a concentration of 0.01 % by weight.

### (3) Production of Protecting Layers

The same method as in the item (1) Production of Dielectric Layer described above was used to produce a bottom protecting layer of 14 mm × 74 mm × 50 µm thickness and a top protecting layer of 14 mm × 67 mm × 50 µm thickness, which were each composed of the polyether type urethane elastomer.

### (4) Production of Adhesive Layer

A machine AWATORI RENTARO (model No.: ARE-310, manufactured by Thinky Corp.) was used to mix 50 parts by weight of an adhesive (SK DYNE 1720, manufactured by Soken Chemical & Engineering Co., Ltd.) with 50 parts by weight of methyl ethyl ketone (MEK) and 2 parts by weight of a curing agent (at 2000 rpm for 120 seconds), and degas the resultant (at 2000 rpm for 120 seconds) to yield a mixture. Next, an applicator was used to form the resultant into a film having a wet film thickness of 100 µm onto a PET film (50E-0010KF, manufactured by Fujimori Kogyo Co., Ltd.) the top surface of which had been subjected to releasing treatment. Thereafter, a blower type oven was used to cure the film at 100 °C for 30 minutes to produce an adhesive layer having a thickness of 25 µm after the curing.

### (5) Manufacture of sensor element

The sensor element was manufactured through the steps illustrated in FIGS. 5A to 5D.

First, a mask (not illustrated) was prepared by forming an opening having a predetermined shape in the PET film which had been subjected to the release treatment, and attached to one side (surface) of a bottom protective layer 25B produced in the step (3).

The mask has the opening corresponding to the bottom electrode layer and the bottom conducting wire, and a size of the opening for the bottom electrode layer was 10 mm (width) × 60 mm (length) and a size thereof for the bottom conducting wire was 5 mm (width) × 10 mm (length).

After that, 7.2 g of the carbon nanotube dispersion liquid prepared in the step (2) was applied from a distance of 10 cm with an air brush, and dried at 100 °C for 10 minutes, whereby a bottom electrode layer 22B and a bottom conducting wire 23B were formed. After that, the mask was removed (refer to FIG. 5A).

After that, a dielectric layer 21 produced in the step (1) was attached and laminated on the bottom protective layer 25B so as to cover the whole of the bottom electrode layer 22B and a part of the bottom conducting wire 23B.

Furthermore, a top electrode layer 22A and a top conducting wire 23A were formed on a top side of the dielectric layer 21 by the same method as that used for forming the bottom electrode layer 22B and the bottom conducting wire 23B (refer to FIG. 5B).

After that, a top protective layer 25A produced in the step (3) was laminated with a laminator on the top side of the dielectric layer 21 having the top electrode layer 22A and the top conducting wire 23A so as to cover the whole of the top electrode layer 22A and a part of the top conducting wire 23A.

Furthermore, a top connecting portion 24A and a bottom connecting portion 24B were formed by attaching copper foil to ends of the top conducting wire 23A and the bottom conducting wire 23B, respectively (refer to FIG. 5C). After that, a lead wire 29 serving as an external conducting wire was fixed with solder to each of the top connecting portion 24A and the bottom connecting portion 24B.

Subsequently, a PET film 27 for reinforcement having a thickness of 100 µm was attached to portions of the top connecting portion 24A and the bottom connecting portion 24B positioned on the bottom protective layer 25B through an acrylic adhesive tape (having a thickness of 0.5 mm) (Y-4905 manufactured by 3M Company) 26.

Finally, an adhesive layer 28 produced in the step (4) was laminated to the bottom surface of the bottom protective layer 25B with a handroller, whereby a sensor element 222 was completed (refer to FIG. 5D).

In the sensor element A manufactured in the present Example, the dielectric layer 21, the top electrode layer 22A, and the bottom electrode layer 22B correspond, respectively, to the first dielectric layer, the first electrode layer, and the second electrode layer.

### Production of Sensor Device

The sensor element 222 manufactured through the items (1) to (5) was connected through lead wires to an LCR meter (LCR HiTESTER 3522-50, manufactured by Hioki E. E. Corp.) to manufacture a sensor device.

In order to inspect the action of the manufactured sensor device, Examples 1 to 4 were performed.

### Example 1: Measurement of Bending and Stretching of Elbow

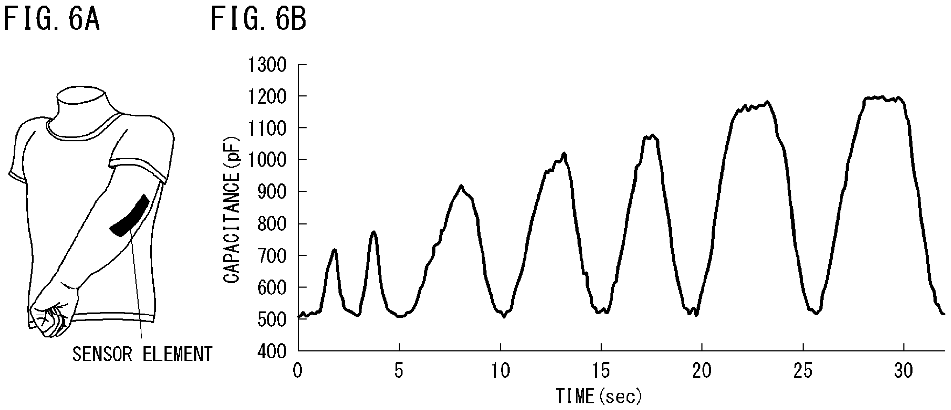

As illustrated in FIG. 6A, the sensor element 222 was bonded to a region of an examinee's left arm elbow to interpose the adhesive layer 28 therebetween.

Thereafter, in the state that the sensor element was bonded thereto, he/she bent and stretched his/her left elbow to make the bending quantity thereof gradually larger. During this period, a change in the capacitance was measured. The result is shown in FIG. 6B.

As shown in the graph of FIG. 6B, it has been made evident that as the bending quantity becomes gradually larger in the bending and stretching motion of the elbow joint, the capacitance becomes gradually larger.

### Example 2: Measurement of Respirations

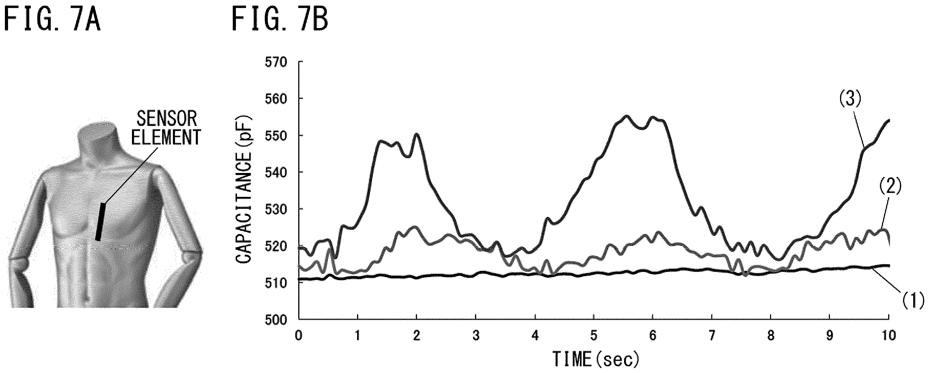

As illustrated in FIG. 7A, the sensor element 222 was bonded to an examinee's left breast to interpose the adhesive layer 28 therebetween.

Thereafter, in the state that the sensor element was bonded thereto, he/she made the following three actions: (1) he/she stopped breathing for 10 seconds; (2) he/she breathed naturally for 10 seconds; and (3) he/she breathed deeply for 10 seconds. At the time of each of the actions, the capacitance was measured. The result is shown in FIG. 7B.

As shown in the graph of FIG. 7B, it has been made evident that according to the action (1), the capacitance hardly changes, and according to the actions (2) and (3), the capacitance becomes large in accordance with the degree of the breathes. Furthermore, it has been able to be presumed that in the actions (2) and (3), breathes were made about 2.5 times for 10 seconds.

### Example 3: Measurement of Pronounced Sounds

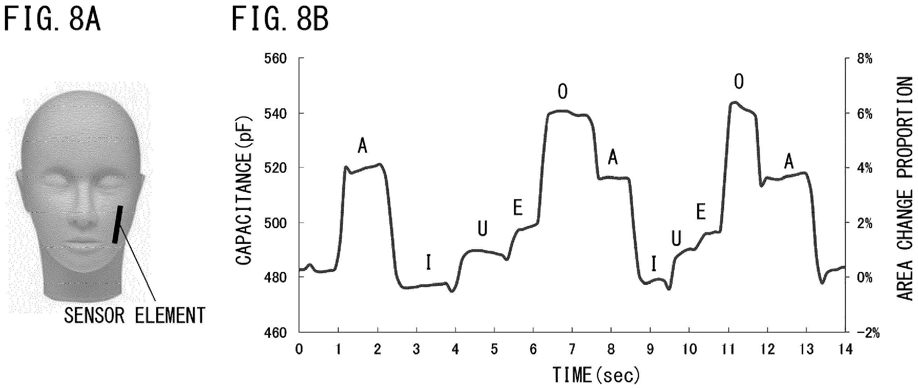

As illustrated in FIG. 8A, the sensor element 22 was bonded to an examinee's left cheek to interpose the adhesive layer 28 therebetween.

Thereafter, in the state that the sensor element was bonded thereto, he/she pronounced "A", "I", "U", "E", and "O", which are Japanese generated sounds. During this period, a change in the capacitance was measured. The result is shown in FIG. 8B.

As shown in the graph of FIG. 8B, it has been made evident that the capacitance is changed in accordance with the species of the pronounced sounds "A", "I", "U", "E", and "O".

### Example 4: Measurement of Pulse Rate (Heart Rate)

(1) Initially, before the sensor element 222 was bonded to an examinee, the capacitance was measured in the state that the sensor element was in the state of being non-elongated.

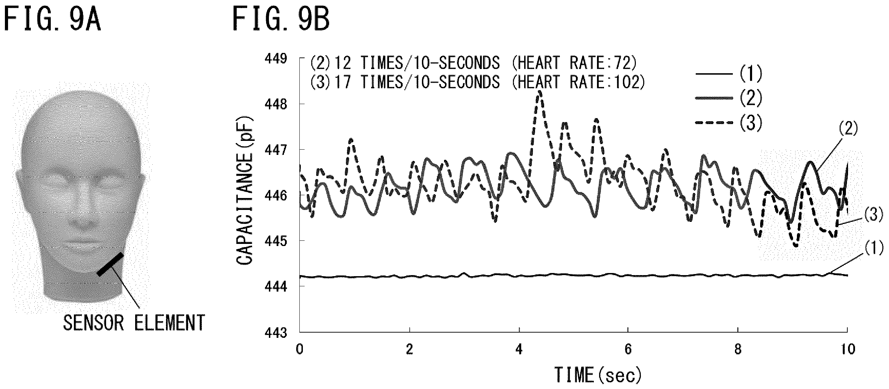

Next, as illustrated in FIG. 9A, the sensor element 222 was bonded onto an examinee's carotid artery (region where pulses were felt) to interpose the adhesive layer 28 therebetween.

Thereafter, (2) the capacitance was measured for 10 seconds in the state that he/she was in an ordinary state. Furthermore, he/she was gotten to stamp his/her feet (200 times per 60-seconds) in the state that he/she was sitting on a chair, and (3) after the stamping, he/she regained his/her breaths for 10 seconds. Thereafter, the capacitance was measured for 10 seconds. The result is shown in FIG. 9B.

At the same time when the capacitance was measured, he/she counted his/her pulses for 10 seconds through his/her left wrist. As a result, the number of the pulses was 12 times at the ordinary time (2), and was 17 times at the time (3) after the exercise. These results were consistent with the result in FIG. 9B.

From these matters, it has been understood that the heart rate is measurable from a change in the capacitance.

### Evaluations of Measurement Precision of Sensor Device: Examples 5 to 8

A sensor element B manufactured by a method described below was connected to a measurement instrument using any one of an automatic balanced bridge circuit (LCR meter), an inverting amplifier circuit, a Schmitt trigger oscillation circuit, and a half-wave double voltage rectification circuit to measure the capacitance (or the voltage correlative with the capacitance). On the basis of the measured result, an evaluation was made about the types of the measurement instrument, and an effect of the manner of connecting the sensor device with the measurement instrument onto the precision of the measurement.

### Production of Sensor Element B

The sensor element B was manufactured in the same way as used to manufacture the sensor element A except that the thickness of the dielectric layer was set to 100 µm and the adhesive layer was not formed.

Accordingly, in the sensor element B, a bottom protecting layer (50 µm), a bottom electrode layer, a dielectric layer (100 µm), a top electrode layer, and a top protecting layer (50 µm) were formed in this order from the bottom side to the top side of this element.

In the present evaluation, in the state that the sensor element was put onto a plate made of polypropylene, the sensor element was put onto a desk to which no countermeasures against static electricity was applied, and then the sensor element was connected to each of the measurement instruments. Thereafter, while this state was kept as it was, a measurement was made (ordinary measurement). Moreover, a measurement was made in the state that the top surface of the top protecting layer was touched with three fingers (finger-touched measurement).

The percentage (%) of the absolute value of the difference between a value measured in the ordinary measurement and a value measured in the finger-touched measurement to the value measured in the ordinary measurement was calculated as an accidental error (%) in the measurement instrument.

### Example 5

As the measurement instrument, an automatic balanced bridge circuit (LCR meter: LCR HiTESTER 3522-50, manufactured by Hioki E. E. Corp.) was used. As a probe for the measurement, a four-terminal probe (model No. 9140, manufactured by Hioki E. E. Corp.) was used. This was connected to the sensor element to measure the capacitance.

At this time, a wiring condition that the top electrode layer was connected to its Lo terminal and the bottom electrode layer was connected to its Hi terminal was defined as a forward connection. Conversely, a wiring condition that the top electrode layer was connected to the Hi terminal and the bottom electrode layer was connected to the Lo terminal was defined as a reverse connection. Under the wiring condition of each of the forward connection and the reverse connection, an ordinary measurement and a finger-touched measurement were made. The results are shown in Table 1.

### Example 6

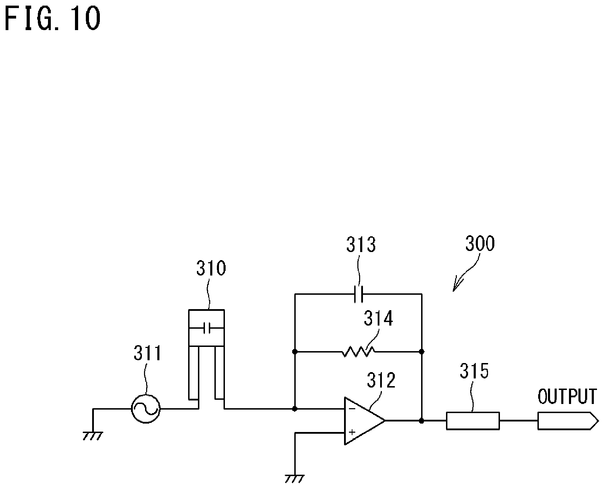

As the measurement instrument, an inverting amplifier circuit 300 as illustrated in FIG. 10 was used. This was connected to a sensor element 310 to measure the capacitance. In the inverting amplifier circuit 300, the oscillation frequency of an AC applying device 311 was set to 5 kHz, the capacitance of a feedback capacity 313, to 329.2 pF, and the resistance value of a feedback resistor 314, to 4.7 MΩ. In FIG. 10, reference number 315 represents a BEF (band elimination filter).

At this time, a wiring condition that the top electrode layer was connected to the AC applying device 311 and the bottom electrode layer was connected to an operating amplifier 312 was defined as a forward connection. Conversely, a wiring condition that the top electrode layer was connected to the operating amplifier 312 and the bottom electrode layer was connected to the AC applying device 311 was defined as a reverse connection. Under the wiring condition of each of the forward connection and the reverse connection, an ordinary measurement and a finger-touched measurement were made. The results are shown in Table 1.

### Example 7

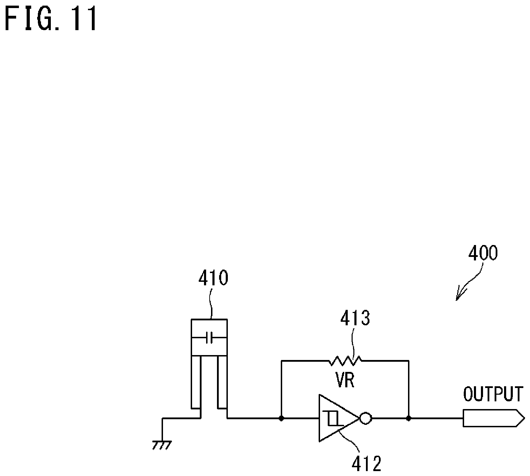

As the measurement instrument, a Schmitt trigger oscillation circuit 400 as illustrated in FIG. 11 was used. This was connected to a sensor element 410 to measure the capacitance through the outputted frequency from a Schmitt trigger 412. In the Schmitt trigger oscillation circuit 400, a variable resistor 413 was adjusted about the resistance value thereof to set the oscillation frequency in a forward connection in any ordinary measurement to 5 kHz.

At this time, a wiring condition that the top electrode layer was earthed and the bottom electrode layer was connected to a Schmitt trigger 412 side was defined as the forward connection. Conversely, a wiring condition that the top electrode layer was connected to the Schmitt trigger 412 side and the bottom electrode layer was earthed was defined as a reverse connection. Under the wiring condition of each of the forward connection and the reverse connection, an ordinary measurement and a finger-touched measurement were made. The results are shown in Table 1.

### Example 8

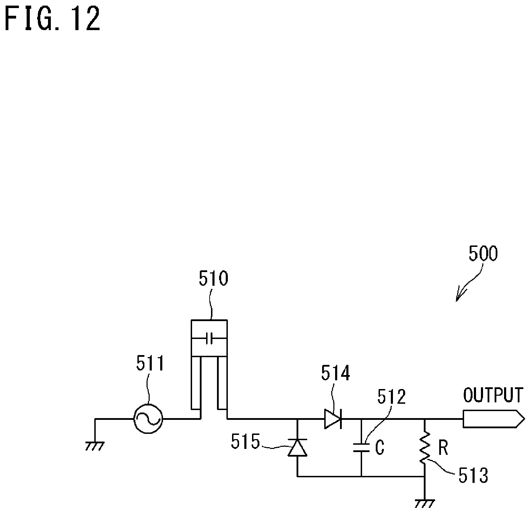

As the measurement instrument, a half-wave double voltage rectification circuit 500 as illustrated in FIG. 12 was used. This was connected to a sensor element 510 to measure the outputted voltage. In the half-wave double voltage rectification circuit 500, the oscillation frequency of an AC applying device 511 was set to 5 kHz, the capacitance of an electrostatic capacitor 512, to 0.1 µF, and the resistance value of a resistor 513, to 470 kΩ. As diodes 514 and 515, Schottky diodes were used.

At this time, a wiring condition that the top electrode layer was connected to the AC applying device 511 and the bottom electrode layer was connected to the output side was defined as a forward connection. Conversely, a wiring condition that the top electrode layer was connected to the output side and the bottom electrode layer was connected to the AC applying device 511 was defined as a reverse connection. Under the wiring condition of each of the forward connection and the reverse connection, an ordinary measurement and a finger-touched measurement were made. The results are shown in Table 1.

**Table 1**

| | Measurement instrument | Wiring condition | Measurement condition | Measured value (pF) | Accidental error (pF) | Accidental error (%) |
|---|---|---|---|---|---|---|
| Example 5 | Automatic balanced bridge circuit (LCR meter) | Forward connection | Ordinary measurement | 256.2 | - | - |
| | | | Finger-touched measurement | 255.5 | -0.7 | 0.27 |
| | | Reverse connection | Ordinary measurement | 256.5 | - | - |
| | | | Finger-touched measurement | 253.8 | -2.7 | 1.05 |
| Example 6 | Inverting amplifier circuit | Forward connection | Ordinary measurement | 224 | - | - |
| | | | Finger-touched measurement | 223 | -1 | 0.45 |
| | | Reverse connection | Ordinary measurement | 224 | - | - |
| | | | Finger-touched measurement | 184 | -40 | 17.86 |
| Example 7 | Schmitt trigger oscillation circuit | Forward connection | Ordinary measurement | 272.5 | - | - |
| | | | Finger-touched measurement | 264.1 | -8.4 | 3.08 |
| | | Reverse connection | Ordinary measurement | 271.0 | - | - |
| | | | Finger-touched measurement | 297.5 | 26.5 | 9.78 |

| | Measurement instrument | Wiring condition | Measurement condition | Measured value (V) | Accidental error (V) | Accidental error (%) |
|---|---|---|---|---|---|---|
| Example 8 | Half-wave double voltage rectification circuit | Forward connection | Ordinary measurement | 1.180 | - | - |
| | | | Finger-touched measurement | 1.176 | -0.004 | 0.34 |
| | | Reverse connection | Ordinary measurement | 1.184 | - | - |
| | | | Finger-touched measurement | 1.214 | 0.03 | 2.53 |

From the results shown in Table 1, it has been made evident that under an accidental error based on the contact of the sensor element with a living body can be decreased by connecting the sensor element with a measurement instrument under a predetermined wring condition. It has been made evident that a large decreasing effect can be obtained according to measurement using, in particular, each of an inverting amplifier circuit, a Schmitt trigger oscillation circuit, and a half-wave double voltage rectification circuit.

In other words, in a measurement through a measurement instrument using each of an inverting amplifier circuit and a half-wave double voltage rectification circuit, it has been made evident that a measurement accidental error can be decreased by connecting the electrode layer made close to and contactable with a living body to an AC applying device (AC-signal generating side). Moreover, in such a measurement through a measurement instrument using a Schmitt trigger oscillation circuit, it has been made evident that a measurement accidental error can be decreased by earthing the electrode layer made close to and contactable with a living body (i.e., connecting this layer to the GND of the measurement instrument).

### REFERENCE SIGNS LIST

- 1: Sensor device
- 2, 2', 222: Sensor element
- 3: Measurement instrument
- 3a, 400: Schmitt trigger oscillation circuit
- 3b: F/V converting circuit
- 4: Indicator
- 4a: Monitor
- 4b: Operating circuit
- 4c: Memory section
- 11,21: Dielectric layer (first dielectric layer)
- 12A, 22A: Top electrode layer (first electrode layer)
- 12B, 22B: Bottom electrode layer (second electrode layer)
- 13A, 23A: Top conducting wire
- 13B, 23B: Bottom conducting wire
- 14A, 24A: Top connection portion
- 14B, 24B: Bottom connection portion
- 15A, 25A, 45A: Top protecting layer
- 15B, 25B, 45B: Bottom protecting layer
- 18, 28: Adhesive layer
- 41A: First dielectric layer
- 41B: Second dielectric layer
- 42A: First electrode layer
- 42B: Second electrode layer
- 42C: Third electrode layer
- 43A: First conducting wire
- 43B: Second conducting wire
- 43C: Third conducting wire
- 44A: First connection portion
- 44B: Second connection portion
- 44C: Third connection portion
- 300: Inverting amplifier circuit
- 500: Half-wave double voltage rectification circuit

## Claims

1. A sensor device (1) comprising:
- a sensor element (2, 2', 222) comprising:
- a sheet-like first dielectric layer (11, 21) comprising an elastomer composition; and
- a first electrode layer (12A, 22A) and a second electrode layer (12B, 22B) each comprising an electro-conductive composition containing carbon nanotubes,
wherein the first and the second electrode layers (12A, 22A, 12B, 22B) are formed on a top surface and a bottom surface of the first dielectric layer (11, 21) respectively, and are at least partially opposed to each other across the first dielectric layer (11, 21),
the at least partially opposed portions of the first and the second electrode layers (12A, 22A, 12B, 22B) constitute a detection portion, and the sensor element (2, 2', 222) is reversibly deformable to change in an area of the top surface and the bottom surface of the first dielectric layer (11, 21); and
a measurement instrument (3) for measuring a change in a capacitance of the detection portion;
the sensor device (1) being adapted to be used in a state of being bonded to a living body and to be utilized to trace a deformation of a surface of the living body,
**characterised in that**:
the sensor element (2, 2', 222) is adapted to be bonded to a living body to make a bottom surface side of the sensor element (2, 2', 222) close to and contactable with the living body, and
wherein the measurement instrument (3) is a measurement instrument (3) for measuring a change in the capacitance using a Schmitt trigger oscillation circuit (400), the first electrode layer formed on the top surface of the first dielectric layer is connected to an oscillation block of the measurement instrument, and the second electrode layer (12B, 22B) formed on the bottom surface of the first dielectric layer (11, 21) is connected to a ground of the measurement instrument (3); or
the measurement instrument (3) is a measurement instrument (3) for measuring a change in the capacitance using an inverting amplifier circuit (300), a half-wave double voltage rectification circuit (500), or an automatic balanced bridge circuit and having an AC applying device, the first electrode layer formed on the top surface of the first dielectric layer is connected to a detecting block of the measurement instrument, and the second electrode layer (12B, 22B) formed on the bottom surface of the first dielectric layer (11, 21) is connected to a side of the measurement instrument (3) where an alternating signal is generated.

2. The sensor device (1) according to claim 1,
wherein the sensor element (2, 2', 222) further comprises a sheet-like second dielectric layer (41B) and a third electrode layer (42C), the second dielectric layer (41B) is laminated on a top side of the first dielectric layer (41A) to cover the first electrode layer (42A) formed over the top surface of the first dielectric layer (41A), and
the third electrode layer (42C) is formed on a top surface of the second dielectric layer (41B) to be at least partially opposed to the first electrode layer (42A) across the second dielectric layer (41B).

3. The sensor device (1) according to any one of claims 1 to 2, further comprising a memory section (4c) for memorizing a measured change in the capacitance.

4. The sensor device (1) according to claim 2 or claim 3,
when claim 3 is dependent upon claim 2,
wherein the first dielectric layer (41A) and second dielectric layer (41B) are elastically deformable such that the area of the top surface and the bottom surface is increased from a non-elongation state by 30 % or more.

## Patentansprüche

1. Sensoreinheit (1), die Folgendes aufweist:
- ein Sensorelement (2, 2', 222), das Folgendes aufweist:
- eine flächenkörperartige erste dielektrische Schicht (11, 21), die eine Elastomer-Zusammensetzung aufweist; und
- eine erste Elektrodenschicht (12A, 22A) sowie eine zweite Elektrodenschicht (12B, 22B), die jeweils eine elektrisch leitfähige Zusammensetzung aufweisen, die Kohlenstoff-Nanoröhren enthält,
- wobei die erste und die zweite Elektrodenschicht (12A, 22A, 12B, 22B) auf einer oberen Oberfläche beziehungsweise einer unteren Oberfläche der ersten dielektrischen Schicht (11, 21) ausgebildet sind und einander zumindest teilweise über die erste dielektrische Schicht (11, 21) hinweg gegenüberliegen,
- wobei die einander zumindest teilweise gegenüberliegenden Bereiche der ersten und der zweiten Elektrodenschicht (12A, 22A, 12B, 22B) einen Detektionsbereich bilden und wobei das Sensorelement (2, 2', 222) reversibel deformierbar ist, so dass es sich in einem Bereich der oberen Oberfläche und der unteren Oberfläche der ersten dielektrischen Schicht (11, 21) ändert; und
- ein Messinstrument (3) zum Messen einer Änderung einer Kapazität des Detektionsbereichs;
- wobei die Sensoreinheit (1) dazu ausgebildet ist, in einem Zustand verwendet zu werden, in dem sie mit einem lebenden Körper verbunden ist, und dazu eingesetzt zu werden, Deformationen einer Oberfläche des lebenden Körpers zu verfolgen,
**dadurch gekennzeichnet,**
**dass** das Sensorelement (2, 2', 222) dazu ausgebildet ist, mit einem lebenden Körper verbunden zu werden, um zu bewirken, dass sich eine untere Oberflächenseite des Sensorelements (2, 2', 222) nahe bei dem lebenden Körper befindet und mit diesem in Kontakt gebracht werden kann, und
- wobei es sich bei dem Messinstrument (3) um ein Messinstrument (3) zum Messen einer Änderung der Kapazität unter Verwendung einer Schmitt-Trigger-Oszillatorschaltung (400) handelt, wobei die erste Elektrodenschicht, die auf der oberen Oberfläche der ersten dielektrischen Schicht ausgebildet ist, mit einem Oszillatorblock des Messinstruments verbunden ist und wobei die zweite Elektrodenschicht (12B, 22B), die an der unteren Oberfläche der ersten dielektrischen Schicht (11, 21) ausgebildet ist, mit der Masse des Messinstruments (3) verbunden ist; oder
- wobei es sich bei dem Messinstrument (3) um ein Messinstrument (3) zum Messen einer Änderung der Kapazität unter Verwendung einer invertierenden Verstärkerschaltung (300), einer Halbwellen-Doppelspannungs-Gleichrichterschaltung (500) oder einer automatisch abgeglichenen Brückenschaltung handelt, das eine einen Wechselstrom anlegende Einheit aufweist, wobei die erste Elektrodenschicht, die auf der oberen Oberfläche der ersten dielektrischen Schicht ausgebildet ist, mit einem Detektionsblock des Messinstruments verbunden ist und die zweite Elektrodenschicht (12B, 22B), die auf der unteren Oberfläche der ersten dielektrischen Schicht (11, 21) ausgebildet ist, mit einer Seite des Messinstruments (3) verbunden ist, an der ein alternierendes Signal erzeugt wird.

2. Sensoreinheit (1) nach Anspruch 1,
- wobei das Sensorelement (2, 2', 222) des Weiteren eine flächenkörperartige zweite dielektrische Schicht (41B) sowie eine dritte Elektrodenschicht (42C) aufweist, wobei die zweite dielektrische Schicht (41B) auf eine obere Seite der ersten dielektrischen Schicht (41A) so laminiert ist, dass sie die über der oberen Oberfläche der ersten dielektrischen Schicht (41A) ausgebildete erste Elektrodenschicht (42A) bedeckt, und
- wobei die dritte Elektrodenschicht (42C) auf einer oberen Oberfläche der zweiten dielektrischen Schicht (41B) so ausgebildet ist, dass sie zumindest teilweise der ersten Elektrodenschicht (42A) über die zweite dielektrische Schicht (41B) hinweg gegenüberliegt.

3. Sensoreinheit (1) nach einem der Ansprüche 1 bis 2,
die des Weiteren einen Speicherbereich (4c) zum Speichern einer gemessenen Änderung der Kapazität aufweist.

4. Sensoreinheit (1) nach Anspruch 2 oder Anspruch 3,
wenn Anspruch 3 von Anspruch 2 abhängig ist,
wobei die erste dielektrische Schicht (41A) und die zweite dielektrische Schicht (41B) derart elastisch deformierbar sind, dass die Fläche der oberen Oberfläche und der unteren Oberfläche in Bezug auf einen nicht gedehnten Zustand um 30 % oder mehr vergrößert wird.

## Revendications

1. Dispositif capteur (1) comprenant :
- un élément capteur (2, 2', 222) comprenant :
- une première couche diélectrique en forme de feuille (11, 21) comprenant une composition élastomère ; et
- une première couche d'électrode (12A, 22A) et une deuxième couche d'électrode (12B, 22B) comprenant chacune une composition électroconductrice contenant des nanotubes de carbone,
dans lequel la première et la deuxième couche d'électrode (12A, 22A, 12B, 22B) sont formées respectivement sur une surface supérieure et une surface inférieure de la première couche diélectrique (11, 21) et sont au moins partiellement opposées l'une à l'autre à travers la première couche diélectrique (11, 21),
les parties au moins partiellement opposées de la première et de la deuxième couche d'électrodes (12A, 22A, 12B, 22B) constituent une partie de détection, et l'élément capteur (2, 2', 222) est déformable de manière réversible afin de changer dans une zone de la surface supérieure et de la surface inférieure de la première couche diélectrique (11, 21) ; et
un instrument de mesure (3) pour mesurer un changement d'une capacité de la partie de détection ;
le dispositif capteur (1) étant adapté à être utilisé dans un état dans lequel il est lié à un corps vivant et à être employé pour suivre une déformation d'une surface du corps vivant,
**caractérisé en ce que** :
l'élément capteur (2, 2', 222) est adapté à être lié à un corps vivant pour rendre un côté de surface inférieure de l'élément capteur (2, 2', 222) proche du corps vivant et apte à entrer en contact avec celui-ci, et
dans lequel l'instrument de mesure (3) est un instrument de mesure (3) pour mesurer un changement de la capacité en utilisant un circuit oscillatoire à bascule de Schmitt (400), la première couche d'électrode formée sur la surface supérieure de la première couche diélectrique est connectée à un bloc oscillatoire de l'instrument de mesure et la deuxième couche d'électrode (12B, 22B) formée sur la surface inférieure de la première couche diélectrique (11, 21) est connectée à une masse de l'instrument de mesure (3) ; ou
l'instrument de mesure (3) est un instrument de mesure (3) pour mesurer un changement de la capacité en utilisant un circuit amplificateur inverseur (300), un circuit de redressement double tension simple alternance (500) ou un circuit en pont équilibré automatique et comportant un dispositif d'application de tension alternative, la première couche d'électrode formée sur la surface supérieure de la première couche diélectrique est connectée à un bloc de détection de l'instrument de mesure et la deuxième couche d'électrode (12B, 22B) formée sur la surface inférieure de la première couche diélectrique (11, 21) est connectée à un côté de l'instrument de mesure (3) où un signal alternatif est généré.

2. Dispositif capteur (1) selon la revendication 1,
dans lequel l'élément capteur (2, 2, 222) comprend en outre une deuxième couche diélectrique en forme de feuille (41B) et une troisième couche d'électrode (42C), la deuxième couche diélectrique (41B) est stratifiée sur un côté supérieur de la première couche diélectrique (41A) pour couvrir la première couche d'électrode (42A) formée sur la surface supérieure de la première couche diélectrique (41A), et la troisième couche d'électrode (42C) est formée sur une surface supérieure de la deuxième couche diélectrique (41B) pour être au moins partiellement opposée à la première couche d'électrode (42A) à travers la deuxième couche diélectrique (41B).

3. Dispositif capteur (1) selon l'une quelconque des revendications 1 à 2, comprenant en outre une section de mémoire (4c) pour mémoriser un changement mesuré de la capacité.

4. Dispositif capteur (1) selon la revendication 2 ou la revendication 3, lorsque la revendication 3 dépend de la revendication 2,
dans lequel la première couche diélectrique (41A) et la deuxième couche diélectrique (41B) sont déformables élastiquement de telle sorte que la zone de la surface supérieure et de la surface inférieure augmente de 30 % ou plus par rapport à un état de non-allongement.
